(19) **Europäisches Patentamt**
**European Patent Office**
**Office européen des brevets**

(11) **EP 2 001 313 B1**

(12) **EUROPEAN PATENT SPECIFICATION**

(45) Date of publication and mention
of the grant of the patent:
**20.08.2014 Bulletin 2014/34**

(51) Int Cl.:
*A23L 3/3508* (2006.01)     *A61L 2/16* (2006.01)
*A23L 3/3463* (2006.01)     *A23L 3/3526* (2006.01)
*A61K 31/195* (2006.01)     *A61K 31/198* (2006.01)
*A61K 35/74* (2006.01)     *A61P 1/12* (2006.01)

(21) Application number: **07727875.2**

(22) Date of filing: **05.04.2007**

(86) International application number:
**PCT/EP2007/053407**

(87) International publication number:
**WO 2007/113333 (11.10.2007 Gazette 2007/41)**

(54) **ANTIMICROBIAL PREPARATIONS**

ANTIMIKROBIELLE PRÄPARATE

PRÉPARATIONS ANTIMICROBIENNES

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **06.04.2006 EP 06112286**

(43) Date of publication of application:
**17.12.2008 Bulletin 2008/51**

(73) Proprietor: **PURAC Biochem BV
4206 AC Gorinchem (NL)**

(72) Inventors:
• **OTTO, Roelf
4201 BC Gorinchem (NL)**
• **RAMIREZ, Aldana, Mariel
6702 AG Wageningen (NL)**

(74) Representative: **Hesselink, Dinah Elisabeth et al
De Vries & Metman
Overschiestraat 180
1062 XK Amsterdam (NL)**

(56) References cited:
**EP-A- 1 629 724     WO-A-2005/105123
WO-A1-2005/112657     JP-A- 8 073 351
US-A- 4 461 777     US-A1- 2001 033 884**

**US-A1- 2003 152 676     US-A1- 2004 127 535
US-B1- 6 881 424**

• **PATENT ABSTRACTS OF JAPAN vol. 2000, no.
11, 3 January 2001 (2001-01-03) & JP 2000 224976
A (SHINKOO GIJUTSU KAIHATSU CENTER:KK;
KIMURA KAZUTAKA), 15 August 2000
(2000-08-15) cited in the application**
• **"Preservation of paste type foods e.g. fish or
artificial meat - by making the paste weakly
alkaline then adding glycine and/or L-serine and
alkali metal salt of organic acid" DERWENT, 1978,
XP002186584**
• **YAMANO TOSHIHIKO ET AL: "Improvement of
the human intestinal flora by ingestion of the
probiotic strain Lactobacillus johnsonii La1"
BRITISH JOURNAL OF NUTRITION, CAMBRIDGE
UNIVERSITY PRESS, CAMBRIDGE, GB, vol. 95,
no. 2, February 2006 (2006-02), pages 303-312,
XP009089924 ISSN: 0007-1145**
• **SREEKUMAR O ET AL: "IMMEDIATE EFFECT OF
LACTOBACILLUS ACIDOPHILUS ON THE
INTESTINAL FLORA AND FECAL ENZYMES OF
RATS AND THE IN VITRO INHIBITION OF
ESCHERICHIA COLI IN COCULTURE" JOURNAL
OF DAIRY SCIENCE, AMERICAN DAIRY SCIENCE
ASSOCIATION, SAVOY, IL, US, vol. 83, no. 5, May
2000 (2000-05), pages 931-939, XP000950235
ISSN: 0022-0302**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Remarks:
The file contains technical information submitted after the application was filed and not included in this specification

Remarks:
The file contains technical information submitted after the application was filed and not included in this specification

**Description**

The invention relates to an antimicrobial preparation and to a process of preserving food. In particular, it relates to a process of preserving food with a combination of at least two amino acids. The invention further relates to the use of such preparations in controlling intestinal flora in humans and animals.

The morbidity and mortality associated with the consumption of food contaminated by infectious and toxin-producing microorganisms should not be underestimated. It is for this reason that the microbial quality and safety of food is a cause of constant concern for food processors, consumers and government agencies. Furthermore the spoilage and putrefaction of a foodstuff by microorganisms can compromise its nutritional value and can lead to substantial economic losses. Microbial spoilage of food results from the uncontrolled proliferation or activities of microorganisms. To prevent this preservation technologies have been developed which ensure the quality and microbiological safety of foods. These technologies are divers and include (i) procedures that prevent access of microorganisms to foods; (ii) procedures that inactivate microorganisms; and (iii) procedures that prevent or slow down the growth of microorganisms. Changes in consumer demands and food legislation actuate food technologists to constantly modify and improve existing food-processing technologies and invent and develop new ones. The trend is to produce foods, which not only look attractive, fresher and more natural but which combine this with the convenience of use such as a long shelf-life and ease of preparation. Furthermore the customer also sets high standards with respect to flavor, texture, appearance and safety. To accomplish all this presents a major challenge for food technologists. With respect to the slowing down or prevention of growth of microorganisms in foods new applications for new and more natural chemical antimicrobials are steadily being made. These new chemical preservatives, however, should satisfy a number of requirements:(i) they should have effective bactericidal or bacteriostatic activity against a wide range of different spoilage organisms and food-borne pathogens; (ii) they should not affect the appearance, taste, flavor or texture of the food; and (iii) they should not be toxic for the consumer. It is known that certain amino acids posses bactericidal or bacteriostatic properties (e.g. Shive,W.; C.G.Skinner (1963) Amino acid analogues. In: Metabolic inhibitors. A comprehensive treatise (Hochster,R.M.; J.H.Quastel eds). Academic Press. New York. Volume I, pp 1 - 73).

Furthermore, many amino acids occur naturally in foods and are generally regarded as save and approved for use in food products. It is exactly for these reasons that some amino acids notably glycine have found commercial application as preservatives.

Processes for preserving foodstuffs with serine were disclosed in US 2,711,976 and US 6,602,532. In US 3,615,703 the flavor of foodstuffs or beverages comprising a fruit or fruit juice, vegetable or vegetable juice, or beer is preserved by hermetically sealing the foodstuff or beverage in a container with lysine, ornithine, histidine, or a salt thereof. GB 1,510,942 discloses a process for the preservation of foodstuffs, particularly those containing 10-60% sugar, e.g. jellies, jams and custards, which are protected against putrefaction by incorporating maltose and glycine in the foodstuffs. Glycine is suitably present in an amount of 0.3 to 2%. Although glycine is now widely used as a commercial preservative it is also recognized that this compound is not a strong preservative and relatively high concentrations are needed to bring about bacterial growth inhibition. These high concentrations, however, create a new set of problems. It is known that glycine, alanine, serine, and threonine all possess, to a different degree a sweet taste, lysine and ornithine both possess bitter and sweet notes whilst arginine is intensely bitter. The impact of amino acids on taste limits the application of these compounds as food preservatives.

In order for glycine to be used more effectively as a food preservative, there is therefore a demand for other substances that might be used in combination with glycine and help to increase its antimicrobial effect. Lee et al. have examined the antimicrobial effect of glycine in combination with hexametaphosphate, EDTA, cholic acid, and glycerol monocaprate on a number of Gram-positive and negative bacterial species (Lee,J.K.; K.Tatsuguchi; M. Tsutsumi; T.Watanabe, J. Food Hyg. Soc. Japan 26: 279 - 284 (1985)). In WO 01/56408 alanine or glycine was used together with 1,5-D-anhydrofructose to obtain a food-keeping agent, which contain highly safe antibacterial substances and thus can improve the keeping qualities of foods without exerting any undesirable effects on the taste or flavor of the foods.

Although processes were disclosed using antimicrobial mixtures consisting of a single amino acid with one or more other non-amino acid compounds (see above), much less is known about the antimicrobial properties of mixtures of amino acids.

JP 2000-224976 discloses a process for the inhibition of microorganisms such as the lactic acid bacterium *Enterococcus faecalis* within a pH region of ≥6.5 of the food, and further hardly damaging the quality of the food using a mixture of calcium lactate and glycine and a salt of some other organic acid. Although in the same application it was also disclosed that part of the glycine can be substituted with alanine, the data show that alanine and mixtures of alanine and glycine are less effective inhibitors of growth of the Gram-positive bacterium *Enterococcus faecalis* than glycine alone. That alanine is able to counteract the inhibitory effect of glycine has not only been shown in case of the closely related Gram-positive bacteria *Enterococcus hirae* and *Lactococcus lactis* but also in the Gram-negative *Escherichia coli* (Snell, E.E.; B.M. Guirard (1943) Proc. Natl. Acad. Sci. USA 29: 66 - 73, Hishinuma, F.; K. Izaki; H. Takahashi (1969) Effects of glycine and D-amino acids on the growth of various micro organisms Agr.Biol.Chem. 33: 1577 - 1586). That one amino acid can cancel out the inhibition exerted by another amino acid is well known and has been observed in a number of Gram-positive and Gram-negative bacterial species e.g. in the Gram-positive bacteria: *Bacillus anthracis* and *Listeria monocytogenes*, and in the Gram-negative bacterium *Escherichia coli* (Gladstone, G.P. (1939) Brit. J. Exp. Pathol. 20: 189 - 200; Friedman, M.E.; W.G.Roessler (1961) J.Bacteriol. 82: 528 - 533; de Felice et al. (1979) Microbiol. Rev. 43: 42 - 58).

In US 2001/033884 a preparation containing glycine and serine is used for preserving food against Gram-positive and Gram-negative bacteria.

Antimicrobial compositions comprising glycine, combined with e.g., alanine or organic acids, are disclosed in US 2004/127535 and EP 16 29 724, respectively.

The abstract of J-53050361 also discloses compositions comprising glycine and serine for adjusting the pH of food.

US 2001/039264 and US 2002/144946 describe other compositions comprising at least 5 or more amino acids for other uses such as reducing blood levels of amino acids associated with severe exercise or reducing fatigue, and for use in hemodialysis, respectively.

It was now found that the combination of glycine and alanine, wherein the alanine is D-alanine surprisingly exerts a synergy in inhibition of Gram-negative bacteria.

To this end the invention pertains to an antimicrobial preparation directed against Gram-negative bacteria comprising at least glycine and alanine.

Thus the present antimicrobial preparation comprising the combination of glycine and alanine, wherein the alanine is D-alanine is particularly suitable for inhibiting the growth of *Escherichia coli* 0157:H7 and *Salmonella* species.

It is stressed that this combination of these two amino acids can be further combined with a third amino acid. It was found that these combinations have an antibacterial effect that is greater than could be expected on the basis of the amino acids in isolation from other amino acids, i.e. show a synergistic effect.

The alanine is D-alanine. Most preferably, the antimicrobial preparation contains DL-alanine. If further other amino acids are present these are most preferably DL-amino acids.

In another aspect, the invention pertains to a method for preserving food against bacteria comprising adding to the food the above-mentioned antimicrobial preparations comprising glycine and alanine.

The method according to the invention is particularly useful for protecting the food against at least one of *Salmonella enterica,* and *Escherichia coli* 0157:H7.

Examples of such food products are meat products (cured and/or uncured, fresh and/or cooked), salads and other vegetable products, drinks and dairy products, semi-processed foods, convenient foods as e.g. ready-to-eat meals and dried food products. The method is of particular interest since some fresh meat products are used for direct consumption (e.g. filet américain, steak tartar, sushi, or carpaccio) without any heat treatment or with heat treatment insufficient to kill bacteria. Other meat products are consumed after application of only partial heat treatment, intentionally applied as e.g. for medium cooked steak or unintentionally applied due to improper preparation or improper handling of the food products.

The antimicrobial preparations of the invention, including preparations comprising glycine/alanine, wherein the alanine is D-alanine can further be applied in other applications as well such as controlling the intestinal flora by inhibiting Gram-negative bacteria such as *Salmonella* and *Escherichia* to provide a selective advantage to Gram-positive bacteria such as Lactobacilli, for instance by administration together with probiotic bacteria.

The following cultures were used in a study: *Escherichia coli* serotype 0157:H7 (ATCC 700728), *Salmonella enterica* (ATCC 13311). All cultures were transferred daily in screw-capped tubes (100 x 16 mm) containing 10 ml brain heart infusion broth (Oxoid, Basingstoke, UK). Cultures were incubated at 30° C without agitation. Brain heart infusion broth was prepared with increasing amounts of the two amino acids. The concentration ranges for the amino acids were as from 0 to 450 mM in 10 50 mM steps. This resulted in 100 different media. The pH of the media was adjusted to 6.1 - 6.2. Media were prepared in 10 ml quantities and sterilized by filtration (Sartorius cellulose nitrate membranes 0.45 $\mu$m pore diameter). 300 $\mu$l of each medium was transferred to a panel of a sterile Bioscreen honeycomb 100 well plate. Well plates were inoculated with 5 $\mu$l of a culture that was grown overnight in brain heart infusion broth using a sterile Hamilton 5 $\mu$l repeating dispenser (Hamilton, Bonaduz, Switserland). Growth rates were determined with a Bioscreen C (Labsystems, Helsinki, Finland) that kinetically measures the development of turbidity by vertical photometry. The plates were incubated for 16 - 24 hours at 37 °C, the optical density of the cultures was measured every 30 minutes at 420 - 580 nm using a wide band filter. The Bioscreen measures at set time intervals the optical density of the cultures. From these data the Bioscreen calculates maximum specific growth rates.

The purpose of further data processing is to ascertain whether two amino acids act independently of each other or whether they stimulate each other in their inhibitory action (synergy) or cancel out each other inhibitory effect (antagonism). When a certain compound has no effect on an organism the specific growth rate of this organism ($\mu$) can be expressed as a function (f) of the growth limiting substrate concentration (s) by for example the Monod equation, which reads: $\mu = \mu_{max} \cdot s / (K_s + s)$, where $\mu_{max}$ represents the maximum specific growth rate, s the standing concentration of the growth limiting substrate in the medium and $K_s$ the substrate concentration where $\mu = 0.5\ \mu_{max}$. However, when the presence of an inhibitor P affects cell growth the function $f$ for $\mu$ must be modified i.e. $\mu = f(s,p)$, where p represents the concentration of inhibitor P. Numerous studies of growth inhibition kinetics of bacteria have shown that many inhibitors behave as non-competitive inhibitors. This implies that only the maximum specific growth rate ($\mu_{max}$) value and not the affinity ($K_s$) is affected. Therefore the specific growth rate in the presence of inhibitor can be written as: $\mu = \mu_i \cdot s / (K_s + s)$, where $\mu_i$ is the maximal specific growth rate in the presence of a inhibitor P. The relationship between $\mu_i$ and $\mu_{max}$ and the concentration of the inhibitor P was describes using the Logistic Dose Response equation, which reads: $\mu_i / \mu_{max} = 1 / (1 + (p / p_{0.5})^b)$ (Jungbauer, A. (2001). The logistic dose response function: a robust fitting function for transition phenomena in life sciences. J. Clinical Ligand Assay 24: 270 - 274). In this equation p represents the concentration of inhibitor P and $p_{0.5}$ the concentration of P where $\mu_i = 0.5\ \mu_{max}$ ; $\mu_{max}$ is the maximum specific growth rate that is the specific growth rate in the absence of inhibitor P, b is a dimensionless quantity, which determines the relationship between $\mu_i$ and p. Combining the Monod and Logistic Dose Response equation it can be written as: $\mu = \mu_{max} (s / K_s + s) / (1 + (p/p_{0.5})^b)$. In batch culture where s is usually many times higher than $K_s$ this equation reduces to $\mu = \mu_{max} / (1 + (p / p_{0.5})^b)$.

When comparing different organisms grown under the same conditions, or the same organism grown under different conditions, it is more meaningful to use relative growth rate, rather than absolute growth rates as standards of comparison. Relative growth rate (O) is the ratio of growth rate ($\mu$) to maximum growth rate ($\mu_{max}$) i.e. $0 = \mu / \mu_{max}$. It can be seen that while $\mu$ and have the dimensions of $(time)^{-1}$ their ratio O is dimensionless, i.e. a pure number. Similarly we can define the relative inhibitor concentration $\epsilon$ as $p / p_{0.5}$. The reduced Monod and Logistic Dose Response equation can now be written as: $O = 1 / (1 + \epsilon^b)$. For two inhibitors X and Y e.g. the following two expressions for O can be defined:

$$O_x = 1 / (1 + \epsilon^{b1}) \text{ and } O_y = 1 / (1 + \epsilon^{b2}).$$

$O_x$ and $O_y$ can be experimentally evaluated by examining the inhibitory effects of either X or Y on the growth rate of the target organism. Knowing the evaluated functions for $O_x$ and $O_y$ the theoretical independent effect is defined as: $O_x.O_y$. The experimentally observed effect of combinations of X and Y on the relative growth rate is defined as $O_{xy}$. The hypothesis that X and Y act independently of each other on a certain organism is mathematically translated to $O_{xy} / O_x.O_y = 1$. Rejection of this hypothesis implies that the combined effect of X and Y is not an additive effect but either synergistic or antagonistic. In case the inhibitors X and Y act synergistically upon the target organism $O_{xy} / O_x.O_y < 1$ (but > 0). In those cases that the combined effect of inhibitors X and Y is antagonistic $O_{xy} / O_x.O_y > 1$. Synergy, independent effect, and antagonism can be visualized in a plot of $O_{xy}$ versus $O_x.O_y$.

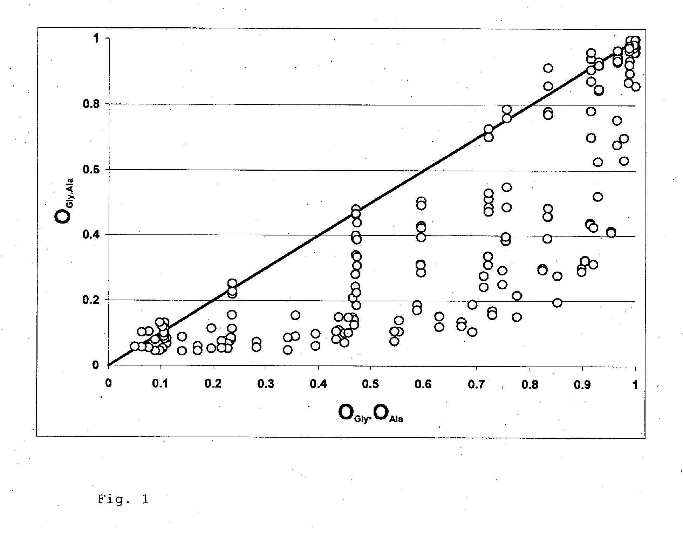

This is exemplified in Fig. 1, wherein a plot is given of $O_{Gly.Ala}$ (experimentally observed relative growth rate) versus $O_{Gly}.O_{Ala}$ (predicted relative growth rate) for *Escherichia coli* O157:H7 (ATCC 700728) showing the synergy in inhibition between Glycine and DL-alanine. The solid line in this graph represents the line where the experimentally observed relative growth rate ($O_{Gly.Ala}$) equals the predicted relative growth rate ($O_{Gly}.O_{Ala}$) and where the Gly and Ala act as independent inhibitors.

The combination of DL-alanine and glycine is particularly effective against *Salmonella enterica* and *Escherichia coli* O157:H7 (synergism)

Competition between *Escherichia coli* O157:H7 (ATCC 700728) and *Lactobacillus plantarum* (DSM 20174) (mixed culture A), and between *Salmonella enterica* (ATCC 13311) and *Lactobacillus plantarum* (DSM 20174) (mixed culture B) was studied in broth cultures. *Escherichia coli, Salmonella enterica,* and *Lactobacillus plantarum* were transferred daily in screw-capped tubes (100 x 16 mm) containing 10 ml brain heart infusion broth (Oxoid, Basingstoke, UK). Cultures were incubated at 30° C without agitation. 500 $\mu$l of an overnight culture of *Escherichia coli* and 5 $\mu$l of a culture of *Lactobacillus plantarum* were transferred to screw capped tubes containing 10 ml of freshly prepared brain heart infusion broth or to 10 ml of brain heart infusion broth containing 200 mM of DL-alanine and 200 mM of glycine or 400 mM of DL-alanine and 400 mM of glycine (mixed culture A, first transfer). 500 $\mu$l of an overnight culture of *Salmonella enterica* and 5 $\mu$l of a culture of *Lactobacillus plantarum* were transferred to screw capped tubes containing 10 ml of freshly prepared brain heart infusion broth or to 10 ml of brain heart infusion broth containing 200 mM of DL-alanine and 200 mM of glycine or 400 mM of DL-alanine and 400 mM of glycine (mixed culture B, first transfer). Both mixed cultures were incubated at 30° C. After 24 hours the cultures were transferred to fresh media (second transfer) and also plated on Violet Red Bile agar (Oxoid, Basingstoke, CM0485) and MRS agar (Oxoid Basingstoke, CM0361). The second transfer was incubated for 24 hours at 30 °C and subsequently plated on Violet Red Bile agar and MRS agar. The results of this analysis, which are summarized in Table 1 demonstrate the competitive advantage of *Lactobacillus plantarum* in a mixed culture with either *Escherichia coli* and *Salmonella enterica* in a medium containing DL-alanine and glycine.

| | Mixed culture A cfu / ml in first transfer | | Mixed culture BI cfu / ml in first transfer | |
|---|---|---|---|---|
| Additions to BHI | E.coli O157:H7 ATCC 700728 | Lb. plantarum DSM 20174 | Salmonella ATCC 13311 | Lb. plantarum DSM 20174 |
| None | 390 . $10^6$ | 268 . $10^6$ | 60 . $10^6$ | 277 . $10^6$ |
| 200 mM Glycine 200 mM DL-Alanine | 25 . $10^6$ | 264 . $10^6$ | 15 . $10^6$ | 660 . $10^6$ |
| 400 mM Gly 400 mM DL-Alanine | 0 | 246 . $10^6$ | 0 | 322 . $10^6$ |

| | Mixed culture A cfu / ml in second transfer | | Mixed culture B cfu / ml in second transfer | |
|---|---|---|---|---|
| Additions to BHI | E.coli O157:H7 ATCC 700728 | Lb. plantarum DSM 20174 | Salmonella ATCC 13311 | Lb. plantarum DSM 20174 |
| None | 291 . $10^6$ | 630 . $10^6$ | 40 . $10^6$ | 780 . $10^6$ |
| 200 mM Glycine 200 mM DL-Alanine | 16 . $10^6$ | 690 . $10^6$ | 0 | 750 . $10^6$ |
| 400 mM Glycine 400 mM DL-Alanine | 0 | 610 . $10^6$ | 0 | 940 . $10^6$ |

Table 1. Colony forming units (cfu) per ml broth in the first and second transfer.

The invention was used for preserving food. He following examples are illustrative of the invention. Stock cultures of *Salmonella typhimurium* ATCC 13311 and *Escherichia coli* O157:H7 ATCC 700728 which were used to inoculate liquid cultures were routinely kept on agar plates containing brain heart infusion (Oxoid CM225, Basingstoke, United Kingdom) fortified with 1.5% agar. Cultures of *Escherichia coli* and *Salmonella typhimurium* were grown in screw-capped tubes (100 x 16 mm) containing 10 ml brain heart infusion broth and incubated overnight at 30 °C. Just prior to the inoculation of the foods the cultures were diluted with a solution containing 0.1% peptone and 0.85% NaCl.

Milk

Sterile non-fat milk was obtained from a local supermarket. Appropriate quantities of amino acids were added.

Pasta (Lasagna)

Ready to Eat Lasagna Bolognese was obtained from a local supermarket and completely homogenized using a tabletop blender. Appropriate quantities of amino acids were added to 500 g of homogenized lasagna. This mixture was vacuum sealed and subsequently irradiated (10 kGray). Irradiated lasagna was stored at -30 °C until further use.

Fresh meat

Freshly cut beef (brisket) containing approximately 20% fat was ground using a Primus MEW 613 Meat grinder (Maschinenfabrik Dornhan, Dornhan, Germany) equipped with a 1/8" grinder plate. The temperature was kept at 10 °C during the entire procedure. Appropriate quantities of amino acids were added to 500 g of ground meat. The meat amino acid mixture was subsequently irradiated (10 kGray). Irradiated meat was stored at -30 °C until further use.

Inoculation of lasagna and fresh meat

Deep frozen turkey ham, lasagna, or fresh meat was thawed overnight at -4 ($\pm$1) °C. 500 g of the still frozen product was quickly cut into 2 - 4 cm pieces and transferred to the bucket of a kitchen food processor (Tefal Kaleo food processor type 67604). 1.5 ml of a suitably diluted bacterial culture was added and the total mix was blended for approximately 30 - 60 seconds. At this stage the temperature of the mix was still below 0 °C. After blending about 25 g thereof was quickly transferred in duplicate to bag filters (Interscience, St Nom, France). Products inoculated with *Escherichia coli* or *Salmonella typhimurium* were incubated for up to two weeks at 12 °C.

Inoculation of milk

Milk was cooled to 10 °C and subsequently inoculated with an appropriately diluted culture of *Salmonella typhimurium.* Inoculated milk samples were incubated at 12 °C.

Microbial analysis

The microbial analysis of the food samples was done as follows: A sealing bag was opened and to this were added 2 times the net weight of sterile dilution fluid (8.5% (w/w) NaCl and 0.1% (w/v) bacteriological peptone). Samples were homogenized for 1 min in a Stomacher 400 lab blender (Seward Medical, London, England) and 50 $\mu$l of the homogenate were plated on a suitable agar medium using an Eddyjet type 1.23 spiral plater (IUL Instruments, Barcelona, Spain). *Escherichia coli* O157:H7 and *Salmonella typhimurium* were plated on VRBG agar (Oxoid, CM0485, Basingstoke, United Kingdom). Plates were incubated for 24 hours at 30 °C and then counted.

Results

The effect of single additions of glycine, DL-alanine and a mixture of glycine (0.67 %) and DL-alanine (0.8 %) on the growth of *Salmonella typhimurium* in milk at 12 °C is given in Table I:

|  | CFU/ml | | | |
| --- | --- | --- | --- | --- |
| Days | 0 | 2 | 4 | 6 |
| Control | $5.10^3$ | $5.10^4$ | $4.10^5$ | $9.10^4$ |
| 0.67% Gly | $5.10^3$ | $5.10^4$ | $1.10^5$ | $8.10^4$ |
| 0.8% Ala | $5.10^3$ | $5.10^4$ | $4.10^5$ | $1.10^5$ |
| 0.67% Gly + 0.8% Ala | $5.10^3$ | $2.10^3$ | $1.10^3$ | $1.10^3$ |

The table shows that in broth cultures glycine to a concentration of 100 mM and DL-alanine to a concentration of 100 mM have little or no effect on the growth rate of *Salmonella typhimurium.* The combinations on the other hand was shown to be very effective in suppressing the growth of this organism in milk.

**Claims**

1. A method for preserving food against Gram-negative bacteria comprising adding to the food an antimicrobial preparation comprising glycine and alanine, wherein the alanine is D-alanine.

2. The method according to claim 1 wherein the antimicrobial preparation comprises DL-alanine.

3. The method according to claim 1 or 2, wherein the food is protected against at least one of Salmonella and Escherichia coli.

4. The method according to claim 3 wherein the food is protected against at least one of Salmonella enterica, Escherichia coli 0157:H7.

5. An antimicrobial preparation comprising two amino acids chosen from at least glycine and alanine for use in controlling the intestinal flora of a human or animal by inhibiting Gram-negative bacteria, wherein the alanine is D-alanine.

6. The antimicrobial preparation of claim 5 comprising a probiotic bacterium and two amino acids chosen from at least glycine and alanine, wherein the alanine is D-alanine.

**Patentansprüche**

1.  Ein Verfahren zur Konservierung von Lebensmitteln gegen Gram-negative Bakterien, das die Zugabe einer antimikrobiellen Zubereitung, umfassend Glycin und Alanin, wobei das Alanin D-Alanin ist, zu dem Lebensmittel umfasst.

2.  Das Verfahren nach Anspruch 1, wobei die antimikrobielle Zubereitung L-Alanin fasst.

3.  Das Verfahren nach Anspruch 1 oder 2, wobei das Lebensmittel gegen mindestens eines von Salmonella und Escherichia coli geschützt ist.

4.  Das Verfahren nach Anspruch 3, wobei das Lebensmittel gegen mindestens eines von Salmonella enterica und Escherichia coli O157:H7 geschützt ist.

5.  Eine antimikrobielle Zubereitung, umfassend zwei Aminosäuren, die aus mindestens Glycin und Alanin ausgewählt sind, zur Verwendung in der Kontrolle der Darmflora eines Menschen oder Tieres durch Hemmen von Gram-negativen Bakterien, wobei das Alanin D-Alanin ist.

6.  Die antimikrobielle Zubereitung nach Anspruch 5, umfassend ein probiotisches Bakterium und zwei Aminosäuren, die aus mindestens Glycin und Alanin ausgewählt sind, wobei das Alanin D-Alanin ist.


**Revendications**

1.  Procédé de préservation d'un aliment contre des bactéries Gram-négative, comprenant l'ajout à l'aliment d'une préparation antimicrobienne comprenant de la glycine et de l'alanine, dans lequel l'alanine est la D-alanine.

2.  Procédé selon la revendication 1, dans lequel la préparation antimicrobienne comprend de la L-alanine.

3.  Procédé selon la revendication 1 ou 2, dans lequel l'aliment est protégé contre au moins une bactérie choisie parmi *Salmonella* et *Escherichia coli.*

4.  Procédé selon la revendication 3, dans lequel l'aliment est protégé contre au moins une bactérie choisie parmi *Salmonella enterica, Escherichia coli* 0157:H7.

5.  Préparation antimicrobie comprenant deux acides aminés choisis parmi au moins la glycine et l'alanine, pour une utilisation dans le contrôle de la flore intestinale d'un humain ou d'un animal par l'inhibition des bactéries Gram-négative, dans laquelle l'alanine est la D-alanine.

6.  Préparation antimicrobienne selon la revendication 5, comprenant une bactérie probiotique et deux acides aminés choisis parmi au moins la glycine et l'alanine, dans laquelle l'alanine est la D-alanine.

Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2711976 A
- US 6602532 B
- US 3615703 A
- GB 1510942 A
- WO 0156408 A
- JP 2000224976 A
- US 2001033884 A
- US 2004127535 A
- EP 1629724 A
- US 2001039264 A
- US 2002144946 A

**Non-patent literature cited in the description**

- Amino acid analogues. **SHIVE,W. ; C.G.SKINNER.** Metabolic inhibitors. A comprehensive treatise. Academic Press, 1963, vol. I, 1-73
- **LEE,J.K. ; K.TATSUGUCHI ; M. TSUTSUMI ; T.WATANABE.** *J. Food Hyg. Soc. Japan,* 1985, vol. 26, 279-284
- **SNELL, E.E. ; B.M. GUIRARD.** *Proc. Natl. Acad. Sci. USA,* 1943, vol. 29, 66-73
- **HISHINUMA, F. ; K. IZAKI ; H. TAKAHASHI.** Effects of glycine and D-amino acids on the growth of various micro organisms. *Agr.Biol.Chem.,* 1969, vol. 33, 1577-1586
- **GLADSTONE,G.P.** *Brit. J. Exp. Pathol.,* 1939, vol. 20, 189-200
- **FRIEDMAN,M.E. ; W.G.ROESSLER.** *J.Bacteriol.,* 1961, vol. 82, 528-533
- **DE FELICE et al.** *Microbiol. Rev.,* 1979, vol. 43, 42-58
- **JUNGBAUER, A.** The logistic dose response function: a robust fitting function for transition phenomena in life sciences. *J. Clinical Ligand Assay,* 2001, vol. 24, 270-274